# FASCICULE DE BREVET EUROPEEN

(11) **EP 2 532 651 B1**
(45) Date de publication et mention de la délivrance du brevet: **05.08.2015**
(21) Numéro de dépôt: 12171118.8
(22) Date de dépôt: 07.06.2012
(51) Int. Cl.: C07D 209/52, A61K 31/403, A61P 25/00

(54) **Procédé de synthèse et forme cristalline du chlorhydrate de 4-{3-[cis-hexahydrocyclopenta[c]pyrrol-2(1h)-yl]propoxy} benzamide ainsi que la base libre associée et les compositions pharmaceutiques qui les contiennent**
Syntheseverfahren und kristalline Form des Chlorhydrats von 4-{3-[cis-hexahydrocyclopenta[c]pyrrol-2(1h)-yl]propoxy}-Benzamid sowie ihre freie Base und die pharmazeutische Zusammensetzungen, die sie enthalten
Synthesis process, and crystalline form of 4-{3-[cis-hexahydrocyclopenta[c]pyrrol-2(1h)-yl]propoxy}benzamide hydrochloride and its free base as well as the pharmaceutical compositions containing them

(30) Priorité: 08.06.2011 FR 1101746
(43) Date de publication de la demande: 12.12.2012
(73) Titulaire: Les Laboratoires Servier, 92284 Suresnes Cedex (FR)
(72) Inventeur: Robert, Nicolas, 76600 Le Havre (FR); Lerestif, Jean-Michel, 76190 Yvetot (FR); Lecouve, Jean-Pierre, 76600 Le Havre (FR); Gaillard, Marina, 45000 Orléans (FR); Meunier, Loïc, décédé (FR); Letellier, Philippe, 45000 Orléans (FR); Boiret, Mathieu, 45000 Orléans (FR)

(56) Documents cités:
- WO-A1-2005/089747
- WO-A1-2010/043787
- WO-A1-2011/070251
- G. ROUSSI, J. ZHANG: "A 3+2 cycloaddition route to N-H pyrrolidines devoid of electron-withdrawing groups", TETRAHEDRON LETTERS, vol. 29, no. 28, 1988, pages 3481-3482, XP002665886,
- BASTIN R J ET AL: "Salt Selection and Optimisation for Pharmaceutical New Chemical Entities", ORGANIC PROCESS RESEARCH AND DEVELOPMENT, CAMBRIDGE, GB, vol. 4, no. 5, 1 January 2000 (2000-01-01) , pages 427-435, XP002228592, DOI: 10.1021/OP000018U

## Description

La présente invention concerne un procédé de synthèse industriel du chlorhydrate de 4-{3-[*cis*-hexahydrocyclopenta[*c*]pyrrol-2(1*H*)-yl]propoxy}benzamide de formule (I) :

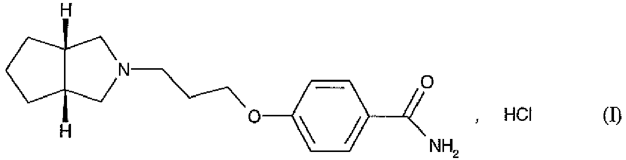

La présente invention concerne également la forme cristalline I du chlorhydrate de 4-{3-[*cis*-hexahydrocyclopenta[*c*]pyrrol-2(1*H*)-yl]propoxy}benzamide, son procédé de préparation ainsi que les compositions pharmaceutiques qui la contiennent.

Par ailleurs, la forme cristalline I de la base libre du composé de formule (I) est également obtenue selon le procédé de l'invention et fait partie intégrante de l'invention, ainsi que les compositions pharmaceutiques qui la contiennent.

Le 4-{3-[*cis*-hexahydrocyclopenta[*c*]pyrrol-2(1*H*)-yl]propoxy}benzamide présente la particularité d'interagir avec les systèmes histaminergiques centraux in vivo. Ces propriétés lui confèrent une activité dans le système nerveux central et plus particulièrement dans le traitement des déficits cognitifs associés au vieillissement cérébral et aux maladies neurodégénératives.

Le 4-{3-[*cis*-hexahydrocyclopenta[*c*]pyrrol-2(1*H*)-yl]propoxy}benzamide, sa préparation sous forme d'oxalate et son utilisation en thérapeutique ont été décrits dans la demande de brevet WO2005/089747.

Compte-tenu de l'intérêt pharmaceutique de ce composé, il était important de pouvoir y accéder avec un procédé de synthèse performant, facilement transposable à l'échelle industrielle, conduisant au chlorhydrate de 4-{3-[*cis*-hexahydrocyclopenta[*c*]pyrrol-2(1*H*)-yl]propoxy}benzamide avec un bon rendement et une excellente pureté.

Il était également important de pouvoir accéder au chlorhydrate de 4-{3-[*cis-*hexahydrocyclopenta[*c*]pyrrol-2(1*H*)-yl]propoxy}benzamide sous une forme cristalline bien définie, parfaitement reproductible et présentant des caractéristiques intéressantes de filtration et de facilité de formulation.

La demande de brevet WO2005/089747 décrit l'accès à l'oxalate de 4-{3-[*cis-*hexahydrocyclopenta[*c*]pyrrol-2(1*H*)-yl]propoxy}benzamide en trois étapes à partir du 4-hydroxybenzonitrile, lequel subit une réaction de O-alkylation avant d'être couplé à un noyau de type octahydracyclopenta[c]pyrrole pour former le 4-{3-[*cis-*hexahydrocyclopenta[*c*]pyrrol-2(1*H*)-yl]propoxy}benzonitrile. Ce dernier composé est finalement soumis à une hydrolyse basique pour conduire au 4-{3-[*cis-*hexahydrocyclopenta[*c*]pyrrol-2(1*H*)-yl]propoxy}benzamide, lequel est cristallisé sous forme d'oxalate. Le rendement de ces trois étapes est de 46,6%.

La présente invention concerne un nouveau procédé de synthèse industriel qui conduit au chlorhydrate de 4-{3-[*cis*-hexahydrocyclopenta[*c*]pyrrol-2(1H)-yl]propoxy}benzamide avec une pureté satisfaisante sur le plan pharmaceutique et un rendement performant sur le plan industriel. Grâce à ce procédé, il est possible de garantir un taux d'impuretés génotoxiques très bas, compatible avec les exigences réglementaires.

Plus spécifiquement, la présente invention concerne un procédé de synthèse industriel du composé de formule (I) : 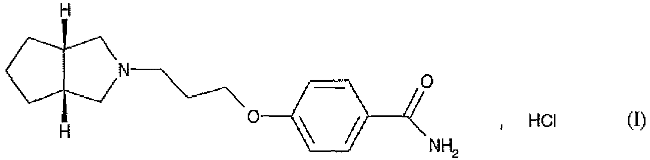 caractérisé en ce que le composé de formule (II) : 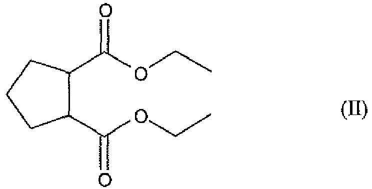 réagit avec l'ammoniaque à une température supérieure à 100°C pour former le composé de formule (III) : 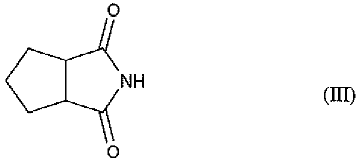 qui est réduit pour conduire à l'amine bicyclique de formule (IV) : 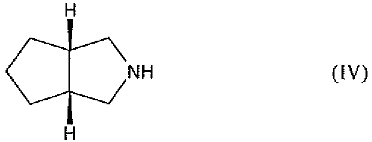 ce dernier composé est soumis par la suite :
- soit à une réaction de couplage en condition basique dans un milieu polaire avec un composé de formule (V) : 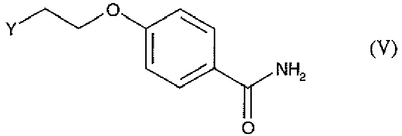 dans laquelle Y représente un -CH₂-Hal dans lequel Hal est un halogène, ou un groupement -CH₂-OSO₂-R dans lequel R est un groupe (C₁-C₆)-alkyl ou un groupe -C₆H₄-CH₃,
- soit à une amination réductrice en milieu acide avec un composé de formule (V'): 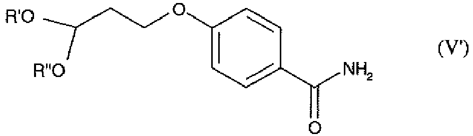 dans laquelle R' et R" représentent indépendamment l'un de l'autre un groupe (C₁-C₆)-alkyl, ou bien R' et R" forment ensemble un groupe -(CH₂)ₙ- où n=2-3, ou bien l'un groupements R' ou R" représentent un atome d'hydrogène et l'autre un groupe (C₁-C₆)-alkyl,
- soit à une amination réductrice avec un composé de formule (V") : 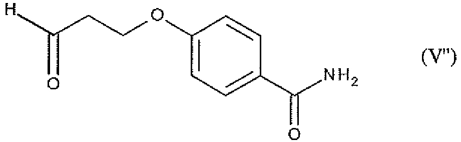 pour conduire à la base libre du composé de formule (I), laquelle est mise en présence d'acide chlorhydrique pour former le composé de formule (I) que l'on isole sous la forme d'un solide.

Dans un mode de réalisation préféré de l'invention, le mélange réactionnel obtenu à l'issue de la réaction du composé de formule (II) avec l'ammoniaque est soumis à une pyrolyse. La pyrolyse en question se fait préférentiellement à une température supérieure ou égale à 200°C, et plus préférentiellement encore à une température supérieure ou égale à 280°C.

La transformation du composé de formule (III) en composé de formule (IV) se fait avantageusement en présence d'hydrogène et d'un catalyseur métallique.

Le composé de formule (V) préféré est le 4-(3-chloropropoxy)benzamide.

La réaction de couplage du composé de formule (IV) avec le composé de formule (V) se fait préférentiellement en présence d'un carbonate, d'une amine ou d'un hydroxyde. Parmi les carbonates, les amines et les hydroxydes préférés, on peut citer le carbonate de potassium, le carbonate de césium, la triéthylamine, la pyridine, l'hydroxyde de potassium, l'hydroxyde de sodium et l'hydroxyde de lithium. Plus préférentiellement encore, la réaction de couplage du composé de formule (IV) avec le composé de formule (V) se fait en présence de carbonate de potassium ou de triéthylamine. De plus, cette réaction est réalisée avantageusement dans un milieu polaire constitué d'un ou plusieurs solvants polaires choisis parmi l'eau, les alcools, les cétones, les éthers, les amides, le DMSO et l'acétonitrile. Les alcools préférés sont le méthanol, l'éthanol, l'isopropanol et le butanol. Parmi les solvants préférés, on retiendra également l'acétone et la méthyléthylcétone pour les cétones, le tétrahydrofurane, le méthyltétrahydrofurane, et le cyclopentylméthyléther pour les éthers, ainsi que la *N*-méthyl-2-pyrrolidone pour les amides. Plus préférentiellement encore, la réaction de couplage du composé de formule (IV) avec le composé de formule (V) est réalisée dans un mélange eau/acétonitrile ou un mélange eau/isopropanol.

Dans le cas d'une amination réductrice en milieu acide du composé de formule (IV) avec un composé de formule (V'), ce dernier est préférentiellement le 4-(3,3-diéthoxypropoxy)benzamide.

Par ailleurs, l'étape de salification de la base libre du composé de formule (I) en présence d'acide chlorhydrique a lieu préférentiellement dans un solvant choisi parmi l'eau, l'acétone ou un alcool. Les alcools préférés sont le méthanol, l'éthanol et l'isopropanol. L'acétone et l'isopropanol sont plus particulièrement préférés pour cette étape de salification.

De manière alternative, le composé de formule (I) isolé à l'issue de l'étape de salification est soumis à une recristallisation.

Il est important de souligner que ce procédé de synthèse permet d'obtenir exclusivement le composé 4-{3-[*cis*-hexahydrocyclopenta[*c*]pyrrol-2(1*H*)-yl]propoxy}benzamide avec un rendement satisfaisant sur le plan industriel, et non pas son homologue *trans.* Outre cet avantage, il permet de maintenir les taux d'impuretés génotoxiques présentes dans les lots (en particulier du 4-(3-chloropropoxy)benzamide) bien au-dessous du seuil réglementaire.

Les composés de formule (V) dans lequel Y représente un groupement -CH₂-OSO₂-R dans lequel R est un groupe (C₁-C₆)-alkyl ou un groupe -C₆H₄-CH₃ et les composés de formule (V') sont nouveaux et utiles en tant qu'intermédiaires de synthèse du composé de formule (I). Le composé de formule (V") est également utile en tant qu'intermédiaire de synthèse du composé de formule (I).

L'invention s'étend également à la forme cristalline I du chlorhydrate de 4-{3-[*cis-*hexahydrocyclopenta[*c*]pyrrol-2(1*H*)-yl]propoxy}benzamide obtenue selon le procédé précédemment décrit. Cette forme cristalline est bien définie, parfaitement reproductible et présente de ce fait des caractéristiques intéressantes de filtration, de séchage, de stabilité et de facilité de formulation.

La forme cristalline I du composé de formule (I) est caractérisée par un diagramme de diffraction X sur poudre présentant les raies de diffraction suivantes (angle de Bragg 2 thêta, exprimé en degrés ±0,2°) : 16,97°, 17,84°, 18,90°, 20,32°, 23,87°, 27,10°, 27,86° et 30,34°.

Plus spécifiquement, la forme cristalline I du chlorhydrate de 4-{3-[*cis-*hexahydrocyclopenta[*c*]pyrrol-2(1*H*)-yl]propoxy}benzamide est caractérisée par le diagramme de diffraction X sur poudre ci-dessous, mesuré à l'aide d'un diffractomètre PANalytical X'Pert Pro MPD avec un détecteur X'Celerator, et exprimé en termes de position de raie (angle de Bragg 2 thêta, exprimé en degrés ±0,2°) et de distance inter-réticulaire d (exprimée en Å) :

| **Raie n°** | **Angle 2-thêta (degrés)** | **Distance inter-réticulaire (Å)** |
|---|---|---|
| 1 | 16,97 | 5,219 |
| 2 | 17,84 | 4,967 |
| 3 | 18,90 | 4,690 |
| 4 | 20,32 | 4,366 |
| 5 | 23,87 | 3,724 |
| 6 | 27,10 | 3,288 |
| 7 | 27,86 | 3,200 |
| 8 | 30,34 | 2,943 |

En outre, la forme I du chlorhydrate de 4-{3-[*cis*-hexahydrocyclopenta[*c*]pyrrol-2(1*H*)-yl]propoxy}benzamide a été caractérisée par spectroscopie Raman. Des pics significatifs ont été observés aux positions suivantes : 1676 cm⁻¹, 1606 cm⁻¹, 1564 cm⁻¹, 1152 cm⁻¹, 830 cm⁻¹ et 296 cm⁻¹.

Alternativement, la forme I du chlorhydrate de 4-{3-[*cis*-hexahydrocyclopenta[c]pyrrol-2(1*H*)-yl]propoxy}benzamide peut être caractérisée par le diagramme de diffraction X sur poudre comportant les 8 raies significatives présentées précédemment, ainsi que par un spectre Raman présentant un pic significatif à la position 1606 cm⁻¹ ou 1676 cm⁻¹ L'obtention de cette forme cristalline a pour avantage de permettre une filtration particulièrement rapide et efficace, ainsi que la préparation de formulations pharmaceutiques ayant une composition constante et reproductible, ce qui est particulièrement avantageux lorsque ces formulations sont destinées à l'administration orale. De plus, la forme I du chlorhydrate de 4-{3-[*cis*-hexahydrocyclopenta[*c*]pyrrol-2(1*H*)-yl]propoxy}benzamide présente des propriétés de solubilité instantanée remarquables.

La forme ainsi obtenue est suffisamment stable pour autoriser son stockage prolongé sans conditions particulières de température, de lumière, d'humidité ou de taux d'oxygène. Plus précisément, la forme I du chlorhydrate de 4-{3-[*cis*-hexahydrocyclopenta[*c*]pyrrol-2(1*H*)-yl]propoxy}benzamide s'est avérée être très stable sur des durées allant jusqu'à 18 mois dans les conditions suivantes :
- à 25°C avec un taux d'humidité de 60% dans un double-sac de polyéthylène,
- à 30°C avec un taux d'humidité de 65% dans un double-sac de polyéthylène,
- à 30°C avec un taux d'humidité de 75% dans un double-sac de polyéthylène.

Il est également décrit la forme cristalline I de la base libre du 4-{3-[*cis-*hexahydrocyclopenta[*c*]pyrrol-2(1*H*)-yl]prapoxy}benzamide obtenue selon le procédé précédemment décrit. Cette forme cristalline est bien définie et parfaitement reproductible. L'obtention de cette forme et son isolement au cours du procédé de synthèse du chlorhydrate de formule (I) décrit précédemment permettent d'éliminer une grande partie des impuretés génotoxiques présentes dans les lots.

La forme cristalline 1 de la base libre du composé de formule (I) est caractérisée par un diagramme de diffraction X sur poudre présentant les raies de diffraction suivantes (angle de Bragg 2 thêta, exprimé en degrés ±0,2°) : 6,25°, 12,55°, 17,74°, 18,19°, 19,43°, 20,72°, 21,00°, 23,50° et 27,00°.

Plus spécifiquement, la forme cristalline 1 de la base libre du 4-{3-[*cis-*hexahydrocyclopenta[c]pyrrol-2(1*H*)-yl]propoxy}benzamide est caractérisée par le diagramme de diffraction X sur poudre ci-dessous, mesuré à l'aide d'un diffractomètre PANalytical X'Pert Pro MPD avec un détecteur X'Celerator, et exprimé en termes de position de raie (angle de Bragg 2 thêta, exprimé en degrés ±0,2°) et de distance inter-réticulaire d (exprimée en Å) :

| **Raie n°** | **Angle 2-thêta (degrés)** | **Distance inter-réticulaire (Å)** |
|---|---|---|
| 1 | 6,25 | 14,131 |
| 2 | 12,55 | 7,049 |
| 3 | 17,74 | 4,997 |
| 4 | 18,19 | 4,873 |
| 5 | 19,43 | 4,565 |
| 6 | 20,72 | 4,284 |
| 7 | 21,00 | 4,226 |
| 8 | 23,50 | 3,782 |
| 9 | 27,00 | 3,297 |

En outre, la forme I de la base libre du 4-{3-[*cis*-hexahydrocyclopenta[*c*]pyrrol-2(1H)-yl]propoxy}benzamide a été caractérisée par spectroscopie Raman. Des pics significatifs ont été observés aux positions suivantes : 292 cm⁻¹, 618 cm⁻¹, 1045 cm⁻¹, 1483 cm⁻¹, 1568 cm⁻¹, 1683 cm⁻¹.

Alternativement, la forme I de la base libre du 4-13-[*cis*-hexahydrocyclopenta[*c*]pyrrol-2(1*H*)-yl]propoxy}benzamide peut être caractérisée par le diagramme de diffraction X sur poudre comportant les 9 raies significatives présentées précédemment, ainsi que par un spectre Raman présentant un pic significatif à la position 1683 cm⁻¹.

Enfin, la forme I de la base libre du 4-{3-[*cis*-hexahydrocyclopenta[*c*]pyrrol-2(1*H*)-yl]propoxy}benzamide a également été caractérisée par spectroscopie RMN à l'état solide. Des pics significatifs ont été observés à 112,2 ppm, 119,2 ppm, 127,2 ppm, 128,6 ppm, 132,4 ppm, 162,2 ppm et 173,2 ppm. Plus précisément, les spectres ¹³C CP/MAS (Cross Polarization Magic Angle Spinning) présentent les pics suivants (exprimés en ppm ± 0,2 ppm):

| **Pic n°** | **Déplacement chimique (ppm)** | **Pic n°** | **Déplacement chimique (ppm)** |
|---|---|---|---|
| 1 | 173,2 | 10 | 59,7 |
| 2 | 162,2 | 11 | 52,1 |
| 3 | 132,4 | 12 | 44,5 |
| 4 | 128,6 | 13 | 42,8 |
| 5 | 127,2 | 14 | 31,5 |
| 6 | 119,2 | 15 | 30,8 |
| 7 | 112,2 | 16 | 30,2 |
| 8 | 67,1 | 17 | 26,2 |
| 9 | 64,0 | | |

L'étude pharmacologique de la forme I du chlorhydrate du 4-{3-[*cis-*hexahydrocyclopenta[*c*]pyrrol-2(1*H*)-yl]propoxy}benzamide, ainsi que celle de la forme I de sa base libre, ont montré une importante activité sur le système nerveux central qui permet d'établir son utilité dans le traitement des troubles cognitifs et psychocomportementaux associés au vieillissement cérébral et aux maladies neurodégénératives, ainsi que dans le traitement des troubles de l'humeur, du syndrome d'hyperactivité ave déficits attentionnels, de l'obésité et de la douleur. Les maladies neurodégénératives plus particulièrement visées sont la maladie d'Alzheimer, la maladie de Parkinson, la maladie de Pick, la maladie de Korsakoff, les démences à corps de Lewy, les démences frontales et sous-corticales, les démences frontotemporales, et les démences vasculaires.

L'invention s'étend aussi aux compositions pharmaceutiques renfermant comme principe actif la forme cristalline 1 du chlorhydrate de 4-{3-[*cis*-hexahydrocyclopenta[*c*]pyrrol-2(1*H*)-yl]propoxy}benzamide, ou bien la forme cristalline I de sa base libre, avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

La posologie utile est adaptable selon la nature et la sévérité de l'affection, la voie d'administration ainsi que l'âge et le poids du patient. Cette posologie varie de 1 mg à 100 mg par jour en une ou plusieurs prises. De manière préférentielle, la forme cristalline I du chlorhydrate de 4-{3-[*cis*-hexahydrocyclopenta[*c*]pyrrol-2(1*H*)-yl]propoxy}benzamide est administrée aux doses journalières (exprimées en équivalent base) de 2 mg, 5 mg et 20 mg (soit 2,25 mg, 5,63 mg et 22,52 mg du chlorhydrate).

Les exemples ci-dessous illustrent l'invention.

### Préparation 1 : 4-(3-Chloropropoxy)benzamide

Dans un réacteur, sont introduits 10,5 kg de 4-hydroxybenzamide, 10,58 kg de carbonate de potassium et 83 kg d'acétonitrile. Le mélange est agité, puis on y ajoute 24,14 kg d'une solution de 1-bromo-3-chloropropane. Le mélange réactionnel est porté au reflux pendant 4 h. On ajoute de l'eau (105 L) à chaud, puis le mélange est refroidi à 5°C et filtré. Le gâteau est lavé par de l'eau, puis de l'acétonitrile. Le produit du titre est obtenu sous la forme d'une poudre avec un rendement de 82 %.
*Point de fusion* : 144°C

### Préparation 2 : 4-(3-Oxoprapoxy)benzamide

### Stade A: 4-(3,3-Diéthoxypropoxy)benzamide

Dans un ballon, on ajoute 500 mg de 4-hydroxybenzamide, 1,51 g de carbonate de potassium, 10 mL de DMF et 730 mg de 3-chloro-1,1-diéthoxypropane. Le milieu réactionnel est agité à 100°C pendant 18 h, puis on y ajoute 5 mL d'eau. La phase aqueuse est extraite avec de l'acétate d'éthyle, puis les phases organiques sont rassemblées, lavées avec de l'eau et concentrées sous pression réduite. Le produit est obtenu sous forme d'une poudre avec un rendement de 89 % et une pureté chimique de 95 %.
*Point de fusion* : 108°C

### Stade B : 4-(3-Oxopropoxy)benzamide

Dans un ballon, on ajoute 5 g du produit obtenu au stade A, 100 ml de THF et 94 mL d'une solution d'acide chlorhydrique 1N. Le milieu est agité à température ambiante pendant 1h. La phase aqueuse est extraite avec du dichlorométhane, puis les phases organiques sont concentrées sous pression réduite. Le produit est obtenu sous forme d'un solide avec un rendement de 96 % et une pureté chimique de 93 %.

### Exemple 1 : Chlorhydrate de 4-{3-[cis-hexahydrocyclopenta[c]pyrrol-2(1H)-yl]propoxy}benzamide

### Stade A : Tetrahydrocyclopenta[c]pyrrole-1,3(2H,3aH)-dione

Dans un autoclave, charger 1 kg de 1,2-cyclopentanedicarboxylate de diéthyle et 1,02 kg d'ammoniaque à 27 %. Le mélange réactionnel est chauffé en autoclave pendant un minimum de 4 heures à une température de 130 °C. Après refroidissement et décompression à 60 °C, on procède à l'évaporation du solvant. Le résidu subit ensuite une pyrolyse à 280 °C pendant 1 heure. L'imide est purifié par distillation sous vide (4-12 mbars) à une température de 200 °C. Après isolement, le produit du titre est obtenu avec un rendement de 96 %.
*Point de fusion :* 89°C

### Stade B : cis-Octahydrocyclopenta[c]pyrrole

Dans un réacteur, on charge 1 kg d'imide du stade A, 250 g de chromite de cuivre et 2 L de dioxane. Le mélange réactionnel est agité jusqu'à absorption complète d'hydrogène à une température de 265 °C et une pression d'hydrogène de 205 bars. Après refroidissement du réacteur, on réalise la filtration du catalyseur.

Les jus d'hydrogénation sont chargés en décanteur, puis on ajoute 0,37 L d'eau. Le pH est ajusté à un pH inférieur à 3 par ajout d'acide sulfurique 96 %. On soutire la phase aqueuse inférieure. Après l'ajout de 2,5 L d'eau, on élimine le dioxane résiduel par distillation azéotropique avec un suivi par indice de réfraction. On procède ensuite à la mise à pH=13 par ajout de lessive de soude 30 %. Le produit du titre est purifié par distillation azéotropique avec l'eau pour obtenir une solution à 30 % massique avec un rendement de 83%.

### Stade C : 4-{3-[cis-Hexahydrocyclopenta[c]pyrrol-2(1H)-yl]propoxy}benzamide

Dans un réacteur, sont introduits 13,35 kg de 4-(3-chloropropoxy)benzamide obtenu selon la préparation 1, 10,33 kg de carbonate de potassium et 168 kg d'acétonitrile. Le mélange est agité. On charge ensuite 34,74 kg de *cis*-octahydrocyclopenta[*c*]pyrrole en solution aqueuse à 30 %, 26,7 L d'eau. Le mélange réactionnel est porté au reflux jusqu'à consommation totale de la matière première. Puis, on procède à l'ajout d'eau (13,3 L), Le milieu est refroidi à 5°C, avant d'être filtré et lavé par de l'eau. Le produit du titre est obtenu sous la forme d'un solide avec un rendement de 81 % et une pureté chimique de 96%.
**RMN¹H** : δ (600,13 MHz ; dmso-d6 ; 300K) : 7,82 (d, 2H, J=9,0 Hz) ; 7,79 (bs, 1H) ; 7,14 (bs, 1H) ; 6,95 (d, 2H, J=9,0 Hz) ; 4,06 (t, 2H, J=6,5 Hz) ; 2,57 (m, 2H) ; 2,48 (m, 2H) ; 2,44 (bt, 2H, J=6,5 Hz) ; 2,14 (bd, 2H, J=7,5 Hz) ; 1,86 (qt, 2H, J=6,5 Hz) ; 1,65-1,55 (m, 3H) ; 1,45-1,38 (m, 1H) ; 1,37-1,30 (m, 2H)
*avec bs : broad singulet, bd : broad doublet, bt : broad triplet*
*Une caractérisation du produit ainsi formé à l'aide des techniques présentées aux Exemples 6 à 8 a montré que la forme I de la base libre était obtenue.*

### Stade D : Chlorhydrate de 4-{3-[cis-Hexahydrocyclopenta[c]pyrrol-2(1H)-yl]propoxy}benzamide

Dans un réacteur, sont introduits 14,69 kg de 4-{3-[*cis*-hexahydrocyclopenta[*c*]pyrrol-2(1*H*)-yl]propoxy}benzamide et 122 L d'eau. Une solution de 6,81 kg d'acide chlorhydrique à 37 % dans 11,54 L d'eau est également préparée. 13,75 kg de cette solution d'acide sont ajoutés dans le réacteur. Le mélange est agité pendant 1 h à température ambiante, puis 1h30 à 60°C. On filtre à chaud la suspension puis l'on rince le filtre par de l'eau. Sur le filtrat, on effectue ensuite un changement de solvant à volume constant pour obtenir un ratio isopropanol/eau 9/1. Le produit est isolé à 0°C et le précipité obtenu est lavé par de l'isopropanol. Le produit du titre est finalement obtenu avec un rendement de 89 % et une pureté chimique supérieure à 99 %.

*Une caractérisation du produit ainsi formé à l'aide des techniques présentées aux Exemples 4 à 5 a montré que la forme I du chlorhydrate était obtenue.*

### Stade E: Chlorhydrate de 4-{3-[cis-Hexahydrocyclopenta[c]pyrrol-2(1H)-yl]propoxybenzamide

Le sel de chlorhydrate obtenu au Stade D est recristallisé dans un mélange d'isopropanol (264 kg) et d'eau (37,4 L). Le mélange est porté au reflux pendant 45 minutes. La solution est filtrée à chaud, puis l'on rince par de l'isopropanol. La cristallisation est ensuite amorcée à 55°C. Le milieu est maintenu à cette température pendant 40 minutes avant d'être refroidi à 0°C. Au bout de quelques heures, le produit est isolé par filtration. Après un lavage à l'isopropanol, on obtient le produit du titre sous la forme d'une poudre avec un rendement de 93 % et une pureté chimique supérieure à 99 %.
*Point de fusion :* 213-215°C
*Une caractérisation du produit ainsi formé à l'aide des techniques présentées aux Exemples 4 à 5 a montré que la forme I du chlorhydrate était obtenue.*

### Exemple 2 : Chlorhydrate de 4-{3-[cis-hexahydrocyclopenta[c]pyrrol-2(1H)-yl]propoxy}benzamide

### Stade A : Tetrahydrocyclopenta[c]pyrrole-1,3(2H,3aH)-dione

Le protocole est identique à celui décrit au Stade A de l'Exemple 1.

### Stade B : cis-Octahydrocyclopenta[c]pyrrole

Le protocole est identique à celui décrit au Stade B de l'Exemple 1.

### Stade C : 4-{3-[cis-Hexahydrocyclopenta[c]pyrrol-2(1H)-yl]propoxy}benzamide

Dans un réacteur, sont introduits 15,2 kg de 4-(3-chloropropoxy)benzamide, 40,28 kg de *cis*-octahydrocyc1openta[*c*]pyrrole en solution aqueuse à 30 %, 63,84 kg d'eau, 21,48 kg d'isopropanol et 14,39 kg de triéthylamine. Le mélange réactionnel est agité et chauffé à reflux jusqu'à consommation totale de la matière première. Puis, le mélange réactionnel est refroidi à 20 °C, avant d'être filtré et lavé par un mélange d'isopropanol et d'eau. Le produit est obtenu sous la forme d'une poudre avec un rendement de 83 % et une pureté chimique de 97 %.
**RMN¹H** : δ (600,13 MHz ; dmso-d6 ; 300K) : 7,82 (d, 2H, J=9,0 Hz) ; 7,79 (bs, 1H) ; 7,14 (bs, 1H) ; 6,95 (d, 2H, J=9,0 Hz) ; 4,06 (t, 2H, J=6,5 Hz) ; 2,57 (m, 2H) ; 2,48 (m, 2H) ; 2,44 (bt, 2H, J=6,5 Hz) ; 2,14 (bd, 2H, J=7,5 Hz) ; 1,86 (qt, 2H, J=6,5 Hz) ; 1,65-1,55 (m, 3H) ; 1,45-1,38 (m, 1H) ; 1,37-1,30 (m, 2H)
*avec bs : broad singulet, bd : broad doublet, bt : broad triplet*
*Une caractérisation du produit ainsi formé à l'aide des techniques présentées aux Exemples 6 à 8 a montré que la forme I de la base libre était obtenue.*

### Stade D : Chlorhydrate de 4-{3-[cis-hexahydrocyclopenta[c]pyrrol-2(1H)-yl]propmy}benzamide

Dans un réacteur, sont introduits 16,49 kg de 4-{3-[*cis*-hexahydrocyclopenta[*c*]pyrrol-2(1*H*)-yl]propoxylbenzamide, 16,36 kg d'acétone, 6,76 kg d'acide chlorhydrique concentré aqueux et 18,96 kg d'eau. Le mélange est agité et chauffé à 50°C pendant 1h. On réalise ensuite une filtration à chaud du mélange vers un second réacteur contenant 57,67 kg d'acétone et 1,65 kg d'eau. Le mélange est ensuite porté à reflux et on ajoute 73,32 kg d'acétone. On maintient le reflux pendant 10 min, puis on refroidit à 0 °C. Le produit est filtré et le solide obtenu est lavé par de l'acétone. Le produit est obtenu sous forme d'une poudre avec un rendement de 85 % et une pureté chimique supérieure à 99 %.

*Une caractérisation du produit ainsi formé à l'aide des techniques présentées aux Exemples 4 à 5 a montré que la forme I du chlorhydrate était obtenue.*

### Exemple 3 : Chlorhydrate de 4-{3-[cis-hexahydrocyclopenta[c]pyrrol-2(1H)-yl]propoxy}benzamide

### Stade A : Tetrahydrocyclopenta[c]pyrrole-1,3(2H,3aH)-dione

Le protocole est identique à celui décrit au Stade A de l'Exemple 1.

### Stade B : cis-Octahydrocyclopenta[c]pyrrole

Le protocole est identique à celui décrit au Stade B de l'Exemple 1.

### Stade C : Chlorhydrate du cis-octahydrocyclopenta[c]pyrrole

Dans un ballon, on dissout 2 g de *cis*-octahydrocyclopenta[*c*]pyrrole dans 10 mL d'éthanol. La solution est refroidie à 0°C, puis on y ajoute 1,64 mL d'une solution d'acide chlorhydrique concentrée (11 M). Le milieu réactionnel est agité à 20°C pendant 30 minutes avant d'être concentré sous pression réduite. Le mélange réactionnel est agité dans le méthyl-*tert*-butyl-éther à 0°C. Le produit est isolé par filtration sous forme d'un solide avec un rendement de 83 % et une pureté chimique de 99 %.
*Point de fusion :* 126°C

### Stade D : Chlorhydrate de 4-{3-[cis-hexahydrocyclopenta[c]pyrrol-2(1H)-yl]propoxy}benzamide

Dans un réacteur, on introduit 915 mg du composé obtenu au Stade C, 1,65 g de triacétoxyborohydrure de sodium, 45 mL de THF et 7,5 mL de triméthylorthoformate. On ajoute ensuite 1 g du composé obtenu à la préparation 2. Le milieu réactionnel est porté à 40°C pendant 50 minutes, puis refroidi à température ambiante et traité avec une solution saturée de NaHCO₃. La phase aqueuse est extraite avec de l'acétate d'éthyle, puis les phases organiques rassemblées sont lavées avec de l'eau. La phase organique est séchée sur MgSO₄, filtrée puis concentrée sous pression réduite. Le résidu obtenu est mis en suspension dans un mélange isopropanol/eau en présence d'acide chlorhydrique. Le milieu réactionnel est porté à 40°C, puis refroidi à 5°C. Le produit est isolé par filtration sous forme d'un solide avec un rendement de 33 % et une pureté chimique de 98 %.

*Une caractérisation du produit ainsi formé à l'aide des techniques présentées aux Exemples 4 à 5 a montré que la forme I du chlorhydrate était obtenue.*

### Exemple 4 : Forme cristalline du chlorhydrate de 4-{3-[cis-hexahydrocyclopenta[c] pyrrol-2(1H)-yl]propoxy}benzamide

Préalablement à l'enregistrement du diagramme de diffraction X, les échantillons obtenus selon le procédé décrit dans l'un des Exemples 1 à 3 ont été broyés pendant 30 secondes à 30 Hz en présence de 100 µL d'éthanol anhydre pour 200 mg de principe actif dans une jarre de 25 mL en acier inoxydable contenant 2 billes en acier inoxydable.

L'enregistrement des données a été effectué sur un diffractomètre PANalytical X'Pert Pro MPD avec un détecteur X'Celerator dans les conditions suivantes :
- Tension 45 kV, intensité 40 mA,
- Montage thêta/thêta,
- Anode : cuivre,
- Longueur d'onde K alpha-1 : 1,54060 Å,
- Longueur d'onde K alpha-2 : 1,54443 Å,
- Rapport K alpha-2/ K alpha-1 : 0,5
- Mode de mesure : continu de 3° à 55° (angle de Bragg 2 thêta) avec une incrémentation de 0,017°,
- Temps de mesure par pas : 35,53 s.

Le diagramme de diffraction X sur poudre de la forme I du chlorhydrate de 4-{3-[*cis-*hexahydrocyclopenta[*c*]pyrrol-2(1*H*)-yl]propoxy}benzamide ainsi obtenu est exprimé en termes de position de raie (angle de Bragg 2 thêta, exprimé en degrés ±0,2°), de distance inter-réticulaire d (exprimée en Å) et d'intensité relative (exprimée en pourcentage par rapport à la raie la plus intense). Les raies significatives ont été rassemblées dans le tableau suivant :

| **Raie n°** | **Angle 2-thêta (degrés)** | **Distance inter-réticulaire (Å)** | **Intensité relative (%)** |
|---|---|---|---|
| 1 | 16,97 | 5,219 | 9,7 |
| 2 | 17,84 | 4,967 | 21,6 |
| 3 | 18,90 | 4,690 | 100 |
| 4 | 20,32 | 4,366 | 41,8 |
| 5 | 23,87 | 3,724 | 15,4 |
| 6 | 27,10 | 3,288 | 44,7 |
| 7 | 27,86 | 3,200 | 6,6 |
| 8 | 30,34 | 2,943 | 21,7 |

Les paramètres suivants ont ainsi été déterminés :
- maille cristalline monoclinique,
- paramètres de maille : a = 10,6621 Å, b = 10,4945 Å, c = 15,6542 Å, β = 101,949°
- groupe d'espace : P 1 2₁/c 1 (14)
- nombre de molécules dans la maille : 4
- volume de la maille : Vₘₐᵢₗₗₑ = 1713,637 Å³
- densité : d = 1,2590 g/cm³.

### Exemple 5 : Forme cristalline I du chlorhydrate de 4-{3-[cis-hexahydrocyclopenta[c] pyrrol-2(1H)-yl]propoxy}benzamide

La forme I du chlorhydrate de 4-{3-[*cis*-hexahydrocyclopenta[*c*]pyrrol-2(1*H*)-yl]propoxy}benzamide a été caractérisée par spectroscopie Raman. Les spectres ont été enregistrés en mode réflexion (PerkinElmer) et transmission (Cobalt) avec une focalisation laser de 785 et 830 nm respectivement, à l'aide d'un détecteur CCD. Le décalage en longueur d'onde dépend de la matière et lui est caractéristique, ce qui permet une analyse de la composition chimique et de l'agencement moléculaire de l'échantillon étudié. Les spectres sont acquis :
- en réflexion avec une puissance laser de 400 mW, une taille de spot de 100 µm, cinq expositions de cinq secondes et une résolution spectrale de 2 cm⁻¹,
- en transmission avec une puissance laser de 650 mW, une taille de spot de 4 mm, 20 expositions de 3 secondes et une résolution spectrale de 2 cm⁻¹.

La gamme spectrale explorée s'échelonne entre 0 et 3278 cm⁻¹ en mode réflexion et entre 37 et 2400 cm⁻¹ en mode transmission.

Des pics significatifs ont été observés aux positions suivantes : 1676 cm⁻¹, 1606 cm⁻¹, 1564 cm⁻¹, 1152 cm⁻¹, 830 cm⁻¹ et 296 cm⁻¹.

Les exemples 6-8 ne sont pas compris dans l'invention et sont seulement cités pour référence.

### Exemple 6 : Forme cristalline I de la base libre du 4-{3-[cis-hexahydrocyclopenta[c] pyrrol-2(1H)-yl]propoxy}benzamide

L'enregistrement des données a été effectué sur un diffractomètre PANalytical X'Pert Pro MPD avec un détecteur X'Celerator dans les conditions suivantes :
- Tension 45 kV, intensité 40 mA,
- Montage thêta/thêta,
- Anode : cuivre,
- Longueur d'onde K alpha-1 : 1,54060 Å,
- Longueur d'onde K alpha-2 : 1,54443 Å,
- Rapport K alpha-2/ K alpha-1 : 0,5
- Mode de mesure : continu de 3° à 55° (angle de Bragg 2 thêta) avec une incrémentation de 0,017°,
- Temps de mesure par pas : 35,53 s.

Le diagramme de diffraction X sur poudre de la forme I de la base libre du 4-{3-[*cis-*hexahydrocyclopenta[*c*]pyrrol-2(1*H*)-yl]propoxy}benzamide obtenu selon le procédé de l'un des Exemples 1à 3 est exprimé en termes de position de raie (angle de Bragg 2 thêta, exprimé en degrés ±0,2°), de distance inter-réticulaire d (exprimée en Å) et d'intensité relative (exprimée en pourcentage par rapport à la raie la plus intense). Les raies significatives ont été rassemblées dans le tableau suivant :

| **Raie n°** | **Angle 2-thêta (degrés)** | **Distance inter-réticulaire (Å)** | **Intensité relative (%)** |
|---|---|---|---|
| 1 | 6,25 | 14,131 | 6,6 |
| 2 | 12,55 | 7,049 | 16,3 |
| 3 | 17,74 | 4,997 | 100 |
| 4 | 18,19 | 4,873 | 7,3 |
| 5 | 19,43 | 4,565 | 13,3 |
| 6 | 20,72 | 4,284 | 32,2 |
| 7 | 21,00 | 4,226 | 7,7 |
| 8 | 23,50 | 3,782 | 51,4 |
| 9 | 27,00 | 3,297 | 5,9 |

### Exemple 7 : Forme cristalline I de la base libre du 4-{3-[cis-hexahydrocyclopenta[c] pyrrol-2(1H)-yl]propoxy}benzamide

La forme **I** de la base libre du 4-{3-[*cis*-hexahydrocyclopenta[*c*]pyrrol-2(1*H*)-yl]propoxy}benzamide a été caractérisée par spectroscopie Raman. Les spectres ont été enregistrés en mode transmission (Cobalt) avec une focalisation laser de 830 nm à l'aide d'un détecteur CCD. Le décalage en longueur d'onde dépend de la matière et lui est caractéristique, ce qui permet une analyse de la composition chimique et de l'agencement moléculaire de l'échantillon étudié. Les spectres ont été acquis avec une puissance de laser de 650 mW, une taille de spot de 4 mm, vingt expositions de 0,9 seconde et une résolution spectrale de 2 cm⁻¹. La gamme spectrale explorée s'échelonne entre 37 et 2400 cm⁻¹.

Des pics significatifs ont été observés aux positions suivantes : 292 cm⁻¹, 618 cm⁻¹, 1045 cm⁻¹, 1483 cm⁻¹, 1568 cm⁻¹, 1683 cm⁻¹.

### Exemple 8 : Forme cristalline I de la base libre du 4-{3-[cis-hexahydrocyclopenta[c] pyrrol-2(1H)-yl]propoxy}benzamide

La forme I de la base libre du 4-{3-[*cis*-hexahydrocyclopenta[*c*]pyrrol-2(1*H*)-yl]propoxy}benzamide a également été caractérisée par spectroscopie RMN à l'état solide. Les spectres ¹³C RMN à l'état solide ont été enregistrés à température ambiante à l'aide d'un spectromètre Bruker SB Avance avec une sonde de type 4 mm CP/MAS SB VTN dans les conditions suivantes :
- Fréquence: 125,76 MHz,
- Largeur spectrale : 40 kHz,
- Vitesse de rotation de l'échantillon à l'angle magique : 13 kHz,
- Séquence d'impulsion: CP (Cross Polarization) avec découplage SPINAL64 (puissance de découplage de 80 Hz),
- Délai de répétitions : 10 s,
- Temps d'acquisition : 47 ms,
- Temps de contact : 4 ms
- Nombre de scans : 4096.
Les spectres ainsi obtenus ont été référencés par rapport à un échantillon d'adamantane. Les pics observés ont été rassemblés dans le tableau suivant (exprimés en ppm ± 0,2 ppm) :

| **Pic n°** | **Déplacement chimique (ppm)** | **Pic n°** | **Déplacement chimique (ppm)** |
|---|---|---|---|
| 1 | 173,2 | 10 | 59,7 |
| 2 | 162,2 | 11 | 52,1 |
| 3 | 132,4 | 12 | 44,5 |
| 4 | 128,6 | 13 | 42,8 |
| 5 | 127,2 | 14 | 31,5 |
| 6 | 119,2 | 15 | 30,8 |
| 7 | 112,2 | 16 | 30,2 |
| 8 | 67,1 | 17 | 26,2 |
| 9 | 64,0 | | |

### Exemple 9 : Composition pharmaceutique

Formule de préparation pour 1000 comprimés dosés à 5 mg (exprimé en équivalent base) :

| | |
|---|---|
| Composé de l'Exemple 1 (exprimé en équivalent base) | 5 g |
| Amidon de mais | 20 g |
| Maltodextrine | 7,5 g |
| Silice colloïdale | 0,2 g |
| Glycolate d'amidon de sodium | 3 g |
| Stéarate de magnésium | 1 g |
| Lactose | 65 g |

## Revendications

1. Procédé de synthèse industriel du composé de formule (I) : 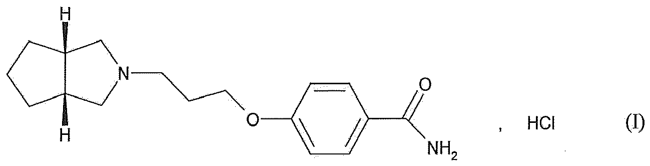 **caractérisé en ce que** le composé de formule (II) : 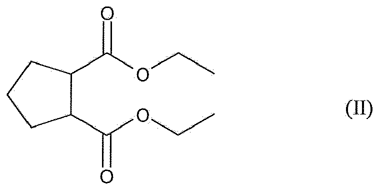 réagit avec l'ammoniaque à une température supérieure à 100°C pour former le composé de formule (III) : 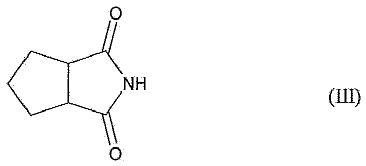 qui est réduit pour conduire à l'amine bicyclique de formule (IV) : 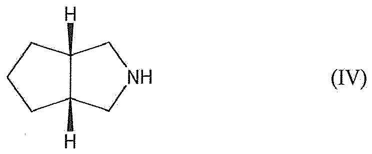 ce dernier composé est soumis par la suite :
- soit à une réaction de couplage en condition basique dans un milieu polaire avec un composé de formule (V) : 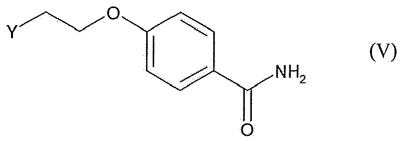 dans laquelle Y représente un -CH₂-Hal dans lequel Hal est un halogène, ou un groupement -CH₂-OSO₂-R dans lequel R est un groupe (C₁-C₆)-alkyl ou un groupe -C₆H₄-CH₃,
- soit à une amination réductrice en milieu acide avec un composé de formule (V') : 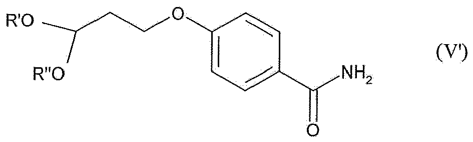 dans laquelle R' et R" représentent indépendamment l'un de l'autre un groupe (C₁-C₆)-alkyl, ou bien R' et R" forment ensemble un groupe -(CH₂)ₙ- où n=2-3, ou bien l'un groupements R' ou R" représentent un atome d'hydrogène et l'autre un groupe (C₁-C₆)-alkyl,
- soit à une amination réductrice avec un composé de formule (V") : 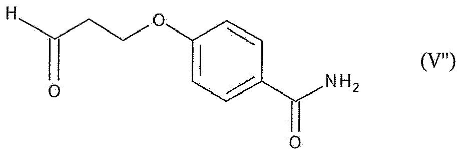 pour conduire à la base libre du composé de formule (I), laquelle est mise en présence d'acide chlorhydrique pour former le composé de formule (I) que l'on isole sous la forme d'un solide.

2. Procédé de synthèse du composé de formule (I) selon la revendication 1 dans lequel le mélange réactionnel obtenu à l'issue de la réaction du composé de formule (II) avec l'ammoniaque est soumis à une pyrolyse.

3. Procédé de synthèse du composé de formule (I) selon la revendication 2 dans lequel la pyrolyse se fait avantageusement à une température supérieure ou égale à 200°C.

4. Procédé de synthèse du composé de formule (I) selon la revendication 2 dans lequel la pyrolyse se fait avantageusement à une température supérieure ou égale à 280°C.

5. Procédé de synthèse du composé de formule (I) selon la revendication 1 dans lequel la transformation du composé de formule (III) en composé de formule (IV) se fait en présence d'hydrogène et d'un catalyseur métallique.

6. Procédé de synthèse du composé de formule (I) selon la revendication 1 dans lequel le composé de formule (V) est le 4-(3-chloropropoxy)benzamide.

7. Procédé de synthèse du composé de formule (I) selon la revendication 1 dans lequel la réaction de couplage du composé de formule (IV) avec le composé de formule (V) se fait en présence d'un carbonate, d'une amine ou d'un hydroxyde.

8. Procédé de synthèse du composé de formule (I) selon la revendication 1 dans lequel la réaction de couplage du composé de formule (IV) avec le composé de formule (V) se fait en présence de carbonate de potassium ou de triéthylamine.

9. Procédé de synthèse du composé de formule (I) selon la revendication 1 dans lequel la réaction de couplage du composé de formule (IV) avec le composé de formule (V) se fait dans un milieu polaire, constitué d'un ou plusieurs solvants polaires choisis parmi l'eau, les alcools, les cétones, les éthers, les amides, le DMSO et l'acétonitrile.

10. Procédé de synthèse du composé de formule (I) selon la revendication 1 dans lequel la réaction de couplage du composé de formule (IV) avec le composé de formule (V) se fait dans un mélange eau/acétonitrile ou un mélange eau/isopropanol.

11. Procédé de synthèse du composé de formule (I) selon la revendication 1 dans lequel l'étape de salification en présence d'acide chlorhydrique a lieu dans un solvant choisi parmi l'eau, l'acétone ou un alcool.

12. Procédé de synthèse du composé de formule (I) selon la revendication 1 dans lequel l'étape de salification en présence d'acide chlorhydrique a lieu dans l'acétone ou l'isopropanol.

13. Procédé de synthèse du composé de formule (I) selon l'une des revendications 1 à 6 dans lequel le composé de formule (I) est soumis à une recristallisation.

14. Composé de formule (V) selon la revendication 1 dans lequel Y représente un groupement -CH₂-OSO₂-R dans lequel R est un groupe (C₁-C₆)-alkyl ou un groupe -C₆H₄-CH₃ utile en tant qu'intermédiaire de synthèse du composé de formule (I).

15. Utilisation d'un composé de formule (V) selon la revendication 1 ou 14 dans la synthèse du composé de formule (I).

16. Composé de formule (V') selon la revendication 1 utile en tant qu'intermédiaire de synthèse du composé de formule (I).

17. Composé de formule (V') selon la revendication 16 qui est le 4-(3,3-diéthoxypropoxy)benzamide.

18. Utilisation d'un composé de formule (V') selon la revendication 16 ou 17 dans la synthèse du composé de formule (I).

19. Utilisation d'un composé de formule (V") selon la revendication 1 dans la synthèse du composé de formule (I).

20. Forme cristalline I du composé de formule (I) selon la revendication 1 : 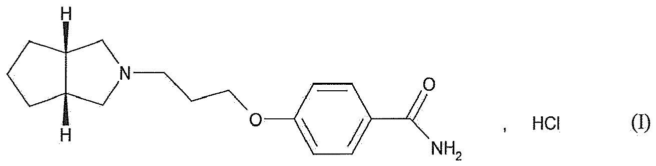 **caractérisée par** un diagramme de diffraction X sur poudre présentant les raies de diffraction suivantes (angle de Bragg 2 thêta, exprimé en degrés ±0,2°) : 16,97°, 17,84°, 18,90°, 20,32°, 23,87°, 27,10°, 27,86° et 30,34°.

21. Forme cristalline I du composé de formule (I) selon la revendication 20 **caractérisée par** le diagramme de diffraction X sur poudre suivant, mesuré sur un diffractomètre PANalytical X'Pert Pro MPD avec un détecteur X'Celerator, et exprimé en termes de position de raie (angle de Bragg 2 thêta, exprimé en degrés ±0,2°), et de distance inter-réticulaire d (exprimée en Å) :
| **Raie n°** | **Angle 2-thêta (degrés)** | **Distance inter-réticulaire (Å)** |
|---|---|---|
| 1 | 16,97 | 5,219 |
| 2 | 17,84 | 4,967 |
| 3 | 18,90 | 4,690 |
| 4 | 20,32 | 4,366 |
| 5 | 23,87 | 3,724 |
| 6 | 27,10 | 3,288 |
| 7 | 27,86 | 3,200 |
| 8 | 30,34 | 2,943 |

22. Forme cristalline I du composé de formule (I) selon la revendication 20 ou 21 **caractérisée par** un spectre Raman présentant un pic significatif à la position 1606 cm⁻¹ ou 1676 cm⁻¹.

23. Forme cristalline I du composé de formule (I) selon l'une des revendications 20 à 22 **caractérisée par** un spectre Raman présentant des pics significatifs aux positions 1676 cm⁻¹, 1606 cm⁻¹, 1564 cm⁻¹, 1152 cm⁻¹, 830 cm⁻¹ et 296 cm⁻¹.

24. Composition pharmaceutique contenant comme principe actif la forme cristalline I du chlorhydrate de 4-{3-[*cis*-hexahydrocyclopenta[*c*]pymol-2(1*H*)-yl]propoxy]benzamide selon l'une des revendications 20 à 23, en combinaison avec un ou plusieurs véhicules inertes, non toxiques et pharmaceutiquement acceptables.

25. Composition pharmaceutique selon la revendication 24 pour son utilisation dans le traitement des troubles cognitifs et psychocomportementaux associés au vieillissement cérébral et aux maladies neurodégénératives, ainsi que pour le traitement des troubles de l'humeur, du syndrome d'hyperactivité avec déficits attentionnels, de l'obésité et de la douleur.

26. Composition pharmaceutique selon la revendication 24 pour son utilisation dans le traitement des troubles cognitifs et psychocomportementaux associés à la maladie d'Alzheimer, la maladie de Parkinson, la maladie de Pick, la maladie de Korsakoff, les démences à corps de Lewy, les démences frontales et sous-corticales, les démences frontotemporales, et les démences vasculaires.

27. Utilisation de la forme cristalline I du chlorhydrate de 4-{3-[*cis-*hexahydrocyclopenta[*c*]pyrrol-2(1*H*)-yl]propoxylbenzamide selon l'une des revendications 20 à 23, pour la fabrication d'un médicament utile pour le traitement des troubles du système histaminergique.

28. Utilisation de la forme cristalline I du chlorhydrate de 4-{3-[*cis-*hexahydrocyclopenta[*c*]pyrrol-2(1*H*)-yl]propoxy}benzamide selon la revendication 27, pour la fabrication d'un médicament destiné au traitement des troubles cognitifs et psychocomportementaux associés au vieillissement cérébral et aux maladies neurodégénératives, ainsi qu'au traitement des troubles de l'humeur, du syndrome d'hyperactivité avec déficits attentionnels, de l'obésité et de la douleur.

29. Utilisation de la forme cristalline I du chlorhydrate de 4-{3-[*cis-*hexahydrocyclopenta[*c*]pyrrol-2(1*H*)-y]]propoxy}benzamide selon la revendication 27, pour la fabrication d'un médicament destiné au traitement des troubles cognitifs et psychocomportementaux associés à la maladie d'Alzheimer, la maladie de Parkinson, la maladie de Pick, la maladie de Korsakoff, les démences à corpsde Lewy, les démences frontales et sous-corticales, les démences frontotemporales, et les démences vasculaires.

30. Forme cristalline I du chlorhydrate de 4-{3-[*cis*-hexahydrocyclopenta[*c*]pyrrol-2(1*H*)-yl]propoxy}benzamide selon l'une des revendications 20 à 23, pour son utilisation dans le traitement des troubles du système histaminergique.

31. Forme cristalline I du chlorhydrate de 4-{3-[*cis*-hexahydrocyclopenta[*c*]pyrrol-2(1*H*)-yl]propoxy}benzamide selon la revendication 30, pour son utilisation dans le traitement des troubles cognitifs et psychocomportementaux associés au vieillissement cérébral et aux maladies neurodégénératives, ainsi qu'au traitement des troubles de l'humeur, du syndrome d'hyperactivité avec déficits attentionnels, de l'obésité et de la douleur.

32. Forme cristalline I du chlorhydrate de 4-{3-[*cis*-hexahydrocyclopenta[*c*]pyrrol-2(1*H*)-yl]propoxy}benzamide selon la revendication 30, pour son utilisation dans le traitement des troubles cognitifs et psychocomportementaux associés à la maladie d'Alzheimer, la maladie de Parkinson, la maladie de Pick, la maladie de Korsakoff, les démences à corps de Lewy, les démences frontales et sous-corticales, les démences frontotemporales, et les démences vasculaires.

## Patentansprüche

1. Verfahren zur industriellen Synthese der Verbindung der Formel (I): 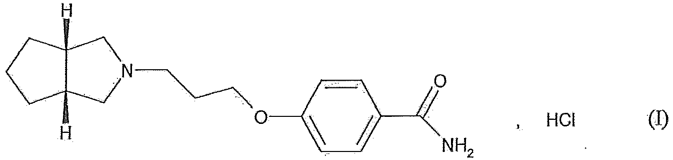 **dadurch gekennzeichnet, dass** die Verbindung der Formel (II): 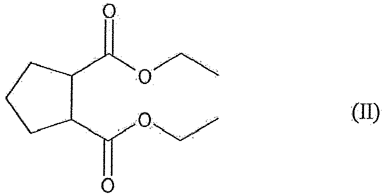 mit Ammoniak bei einer Temperatur von mehr als 100 °C umgesetzt wird zur Bildung der Verbindung der Formel (III): 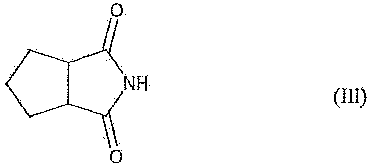 welche reduziert wird zur Bildung des bicyclischen Amins der Formel (IV): 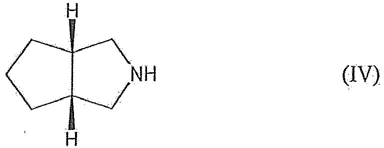 welchletztere Verbindung anschließend:
- entweder einer Kupplungsreaktion unter basischen Bedingungen in einem polaren Lösungsmittel mit einer Verbindung der Formel (V) unterworfen wird: 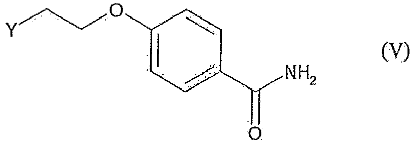 in der Y -CH₂-Hal, worin Hal ein Halogenatom darstellt, oder eine Gruppe -CH₂-OSO₂-R bedeutet, worin R eine (C₁-C₆)-Alkylgruppe oder eine -C₆H₄-CH₃-Gruppe darstellt,
- oder einer reduzierenden Aminierung in saurem Medium mit einer Verbindung der Formel (V') unterworfen wird: 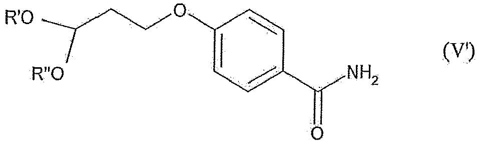 in der R' und R" unabhängig voneinander eine (C₁-C₆)-Alkylgruppe bedeutet oder R' und R" gemeinsam eine Gruppe -(CH₂)ₙ-, worin n = 2-3 darstellt, bilden oder eine der Gruppen R' oder R" ein Wasserstoffatom und die andere eine (C₁-C₆)-Alkylgruppe bedeuten,
- oder einer reduzierenden Aminierung mit einer Verbindung der Formel (V") unterworfen wird: 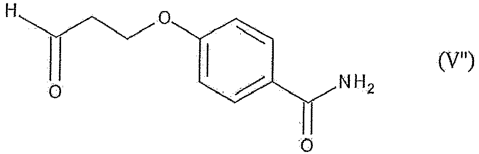 zur Bildung der freien Base der Verbindung der Formel (I), welche der Gegenwart von Chlorwasserstoffsäure ausgesetzt wird zur Bildung der Verbindung der Formel (I), die man in Form eines Feststoffs isoliert.

2. Verfahren zur Synthese der Verbindung der Formel (I) nach Anspruch 1, worin die nach der Umsetzung der Verbindung der Formel (II) mit Ammoniak erhaltene Reaktionsmischung einer Pyrolyse unterworfen wird.

3. Verfahren zur Synthese der Verbindung der Formel (I) nach Anspruch 2, worin die Pyrolyse mit Vorteil bei einer Temperatur von 200 °C oder mehr erfolgt.

4. Verfahren zur Synthese der Verbindung der Formel (I) nach Anspruch 2, worin die Pyrolyse mit Vorteil bei einer Temperatur von 280 °C oder mehr erfolgt.

5. Verfahren zur Synthese der Verbindung der Formel (I) nach Anspruch 1, worin die Umwandlung der Verbindung der Formel (III) in die Verbindung der Formel (IV) in Gegenwart von Wasserstoff und eines Metallkatalysators erfolgt.

6. Verfahren zur Synthese der Verbindung der Formel (I) nach Anspruch 1, worin die Verbindung der Formel (V) 4-(3-Chlorpropoxy)-benzamid ist.

7. Verfahren zur Synthese der Verbindung der Formel (I) nach Anspruch 1, worin die Reaktion der Kupplung der Verbindung der Formel (IV) mit der Verbindung der Formel (V) in Gegenwart eines Carbonats, eines Amins oder eines Hydroxids erfolgt.

8. Verfahren zur Synthese der Verbindung der Formel (I) nach Anspruch 1, worin die Reaktion der Kupplung der Verbindung der Formel (IV) mit der Verbindung der Formel (V) in Gegenwart von Kaliumcarbonat oder Triethylamin erfolgt.

9. Verfahren zur Synthese der Verbindung der Formel (I) nach Anspruch 1, worin die Reaktion der Kupplung der Verbindung der Formel (IV) mit der Verbindung der Formel (V) in einem polaren Lösungsmittel erfolgt, welches durch ein oder mehrere polare Lösungsmittel gebildet wird, ausgewählt aus Wasser, Alkoholen, Ketonen, Ethern, Amiden, DMSO und Acetonitril.

10. Verfahren zur Synthese der Verbindung der Formel (I) nach Anspruch 1, worin die Reaktion der Kupplung der Verbindung der Formel (IV) mit der Verbindung der Formel (V) in einer Wasser/Acetonitril-Mischung oder einer Wasser/Isopropanol-Mischung erfolgt.

11. Verfahren zur Synthese der Verbindung der Formel (I) nach Anspruch 1, worin die Stufe der Salzbildung in Gegenwart von Chlorwasserstoffsäure in einem Lösungsmittel erfolgt, ausgewählt aus Wasser, Aceton oder einem Alkohol.

12. Verfahren zur Synthese der Verbindung der Formel (I) nach Anspruch 1, worin die Stufe der Salzbildung in Gegenwart von Chlorwasserstoffsäure in Aceton oder Isopropanol erfolgt.

13. Verfahren zur Synthese der Verbindung der Formel (I) nach einem der Ansprüche 1 bis 6, worin die Verbindung der Formel (I) einer Umkristallisation unterzogen wird.

14. Verbindung der Formel (V) nach Anspruch 1, worin Y eine Gruppe -CH₂-OSO₂-R bedeutet, worin R eine (C₁-C₆)-Alkylgruppe oder eine Gruppe -C₆H₄-CH₃ bedeutet, nützlich als Zwischenprodukt bei der Synthese der Verbindung der Formel (I).

15. Verwendung einer Verbindung der Formel (V) nach Anspruch 1 oder 14 bei der Synthese der Verbindung der Formel (I).

16. Verbindung der Formel (V') nach Anspruch 1, nützlich als Zwischenprodukt bei der Synthese der Verbindung der Formel (I).

17. Verbindung der Formel (V') nach Anspruch 16, nämlich 4-(3,3-Diethoxypropoxy)-benzamid.

18. Verwendung einer Verbindung der Formel (V') nach Anspruch 16 oder 17 bei der Synthese der Verbindung der Formel (I).

19. Verwendung einer Verbindung der Formel (V") nach Anspruch 1 bei der Synthese der Verbindung der Formel (I).

20. Kristallform I der Verbindung der Formel (I) nach Anspruch 1: 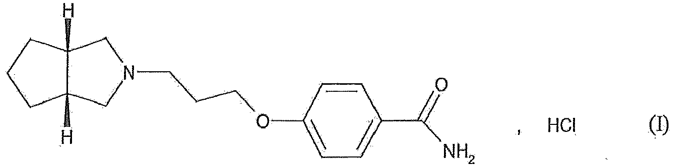 **gekennzeichnet durch** ein Pulverröntgenbeugungsdiagramm mit den folgenden Beugungslinien (Bragg-Winkel 2 Theta ausgedrückt in Grad ±0,2°): 16,97°, 17,84°, 18,90°, 20,32°, 23,87°, 27,10°, 27,86° und 30,34°.

21. Kristallform I der Verbindung der Formel (I) nach Anspruch 20, **gekennzeichnet durch** das folgenden Pulverröntgenbeugungsdiagramm, gemessen mit einem Diffraktometer PANalytical X'Pert Pro MPD mit einem Detektor X'Celerator und ausgedrückt als Linienpositionen (Bragg-Winkel 2 Theta, ausgedrückt in Grad ±0,2°) und Netzebenenabstände d (ausgedrückt in Å):
| **Linie Nr.** | **2 Theta-Winkel (Grad)** | **Netzebenenabstand (Å)** |
|---|---|---|
| 1 | 16,97 | 5,219 |
| 2 | 17,84 | 4,967 |
| 3 | 18,90 | 4,690 |
| 4 | 20,32 | 4,366 |
| 5 | 23,87 | 3,724 |
| 6 | 27,10 | 3,288 |
| 7 | 27,86 | 3,200 |
| 8 | 30,34 | 2,943 |

22. Kristallform I der Verbindung der Formel (I) nach Anspruch 20 oder 21, **gekennzeichnet durch** ein Raman-Spektrum mit einem signifikanten Peak in der Position 1606 cm⁻¹ oder 1676 cm⁻¹.

23. Kristallform I der Verbindung der Forme 1(I) nach einem der Ansprüche 20 bis 22, **gekennzeichnet durch** ein Raman-Spektrum, umfassend signifikante Peaks in den Positionen 1676 cm⁻¹, 1606 cm⁻¹, 1564 cm⁻¹, 1152 cm⁻¹, 830 cm⁻¹ und 296 cm⁻¹.

24. Pharmazeutische Zubereitung enthaltend als Wirkstoff die Kristallform I des Hydrochlorids von 4-{3-[*cis*-Hexahydrocyclopenta[*c*]pyrrol-2-(1*H*)-yl]-propoxy}-benzamid gemäß einem der Ansprüche 20 bis 23 in Kombination mit einem oder mehreren inerten, nichttoxischen und pharmazeutisch annehmbaren Hilfsstoffen.

25. Pharmazeutische Zubereitung nach Anspruch 24 zur Verwendung bei der Behandlung von kognitiven und psychoverhaltensmäßigen Störungen, die mit dem Altern des Gehirns und neurodegenerativen Erkrankungen verknüpft sind, sowie zur Behandlung von Gemütsstörungen, des Hyperaktivitätssyndroms mit Aufmerksamkeitsdefiziten, Fettsucht und Schmerz.

26. Pharmazeutische Zubereitung nach Anspruch 24 zur Verwendung bei der Behandlung von kognitiven und psychoverhaltensmäßigen Störungen, die mit der Alzheimerschen Krankheit, der Parkinsonschen Krankheit, der Pickschen Krankheit, der Korsakoff-Krankheit, Lewy-Körper-Demenzen, frontalen und subkortikalen Demenzen, frontotemporalen Demenzen und vaskulären Demenzen verknüpft sind.

27. Verwendung der Kristallform I des Hydrochlorids von 4-{3[cis-Hexahydrocyclopenta[c]pyrrol-2(1*H*)-yl]-propoxy}-benzamid nach einem der Ansprüche 20 bis 23 für die Herstellung eines Arzneimittels, nützlich zur Behandlung von Störungen des histaminergischen Systems.

28. Verwendung der Kristallform I des Hydrochlorids von *4-{3-[cis-*Hexahydrocyclopenta[*c*]pyrrol-2(1*H*)-yl]-propoxy}-benzamid nach Anspruch 27 für die Herstellung eines Arzneimittels zur Behandlung von kognitiven und psychoverhaltensmäßigen Störungen, die verknüpft sind mit dem Altern des Gehirns und neurodegenerativen Erkrankungen sowie zur Behandlung von Gemütsstörungen, des Hyperaktivitätssyndroms mit Aufmerksamkeitsdefiziten, der Fettsucht und Schmerz.

29. Verwendung der Kristallform I des Hydrochlorids von *4-{3-[cis-*Hexahydrocyclopenta[*c*]pyrrol-2(1*H*)-yl]-propoxy}-benzamid nach Anspruch 27 für die Herstellung eines Arzneimittels zur Behandlung von kognitiven und psychoverhaltensmäßigen Störungen, die verknüpft sind mit der Alzheimerschen Krankheit, der Parkinsonschen Krankheit, der Pickschen Krankheit, der Korsakoff-Krankheit, Lewy-Körper-Demenzen, frontalen und subkortikalen Demenzen, frontotemporalen Demenzen und vaskulären Demenzen.

30. Kristallform I des Hydrochlorids von 4-{3-[*cis*-Hexahydrocyclopenta[*c*]pyrrol-2(1*H*)-yl]-propoxy}-benzamid nach einem der Ansprüche 20 bis 23 zur Verwendung bei der Behandlung von Störungen des histaminergischen Systems.

31. Kristallform I des Hydrochlorids von 4-{3-[*cis*-Hexahydrocyclopenta[*c*]pyrrol-2(1*H*)-yl]-propoxy}-benzamid nach Anspruch 30 zur Verwendung bei der Behandlung von kognitiven und psychoverhaltensmäßigen Störungen, die verknüpft sind mit dem Altern des Gehirns und neurodegenerativen Erkrankungen sowie zur Behandlung von Gemütsstörungen, der Hyperaktivitätssyndroms mit Aufmerksamkeitsdefiziten, Fettsucht und Schmerz.

32. Kristallform I des Hydrochlorids von 4-{3-[*cis*-Hexahydrocyclopenta[*c*]pyrrol-2(1*H*)-yl]-propoxy}-benzamid nach Anspruch 30 zur Verwendung bei der Behandlung von kognitiven und psychoverhaltensmäßigen Störungen, die verknüpft sind mit der Alzheimerschen Krankheit, de Parkinsonschen Krankheit, der Pickschen Krankheit, der Korsakoff-Krankheit, Lewy-Körper-Demenzen, frontalen und subkortikalen Demenzen, frontotemporalen Demenzen und vaskulären Demenzen.

## Claims

1. Process for the industrial synthesis of the compound of formula (I): 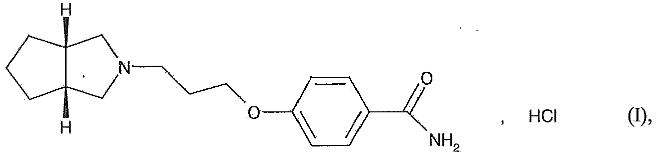 **characterised in that** the compound of formula (II): 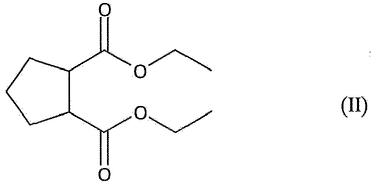 is reacted with ammonia at a temperature greater than 100°C to form the compound of formula (III): 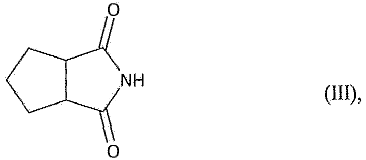 which is reduced to yield the bicyclic amine of formula (IV): 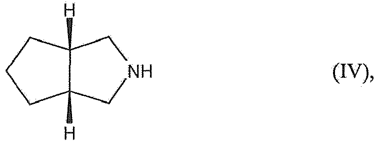 which latter compound is subsequently subjected:
- either to a coupling reaction, under basic conditions in a polar medium, with a compound of formula (V): 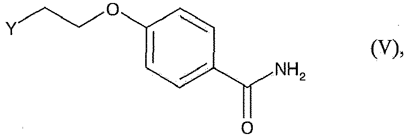 wherein Y represents -CH₂-Hal wherein Hal is a halogen, or a group -CH₂-OSO₂-R wherein R is a (C₁-C₆)alkyl group or a -C₆H₄-CH₃ group,
- or to reductive amination, in an acid medium, with a compound of formula (V'): 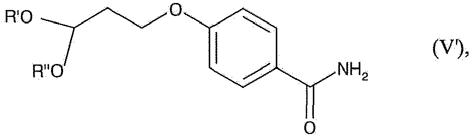 wherein R' and R" represent, each independently of the other, a (C₁-C₆)alkyl group, or R' and R" together form a group -(CH₂)ₙ- wherein n = 2-3, or one of the groups R' and R" represents a hydrogen atom and the other represents a (C₁-C₆)alkyl group,
- or to reductive amination with the compound of formula (V"): 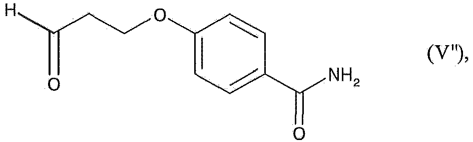 to yield the free base of the compound of formula (I), which is placed in the presence of HCl to form the compound of formula (I), which is isolated in the form of a solid.

2. Process for the synthesis of the compound of formula (I), according to claim 1, wherein the reaction mixture obtained at the end of the reaction of the compound of formula (II) with ammonia is subjected to pyrolysis.

3. Process for the synthesis of the compound of formula (I), according to claim 2, wherein the pyrolysis is advantageously carried out at a temperature greater than or equal to 200°C.

4. Process for the synthesis of the compound of formula (I), according to claim 2, wherein the pyrolysis is advantageously carried out at a temperature greater than or equal to 280°C.

5. Process for the synthesis of the compound of formula (I), according to claim 1, wherein the conversion of the compound of formula (III) into the compound of formula (IV) is carried out in the presence of hydrogen and a metallic catalyst.

6. Process for the synthesis of the compound of formula (I), according to claim 1, wherein the compound of formula (V) is 4-(3-chloropropoxy)benzamide.

7. Process for the synthesis of the compound of formula (I), according to claim 1, wherein the coupling reaction of the compound of formula (IV) with the compound of formula (V) is carried out in the presence of a carbonate, an amine or a hydroxide.

8. Process for the synthesis of the compound of formula (I), according to claim 1, wherein the coupling reaction of the compound of formula (IV) with the compound of formula (V) is carried out in the presence of potassium carbonate or triethylamine.

9. Process for the synthesis of the compound of formula (I), according to claim 1, wherein the coupling reaction of the compound of formula (IV) with the compound of formula (V) is carried out in a polar medium composed of one or more polar solvents selected from water, alcohols, ketones, ethers, amides, DMSO and acetonitrile.

10. Process for the synthesis of the compound of formula (I), according to claim 1, wherein the coupling reaction of the compound of formula (IV) with the compound of formula (V) is carried out in a water/acetonitrile mixture or a water/isopropanol mixture.

11. Process for the synthesis of the compound of formula (I), according to claim 1, wherein the salt formation step in the presence of HCl takes place in a solvent selected from water, acetone and an alcohol.

12. Process for the synthesis of the compound of formula (I), according to claim 1, wherein the salt formation step in the presence of HCl takes place in acetone or isopropanol.

13. Process for the synthesis of the compound of formula (I), according to one of claims 1 to 6, wherein the compound of formula (I) is subjected to recrystallisation.

14. Compound of formula (V) according to claim 1, wherein Y represents a group -CH₂-OSO₂ wherein R is a (C₁-C₆)alkyl group or a -C₆H₄-CH₃ group, for use as a synthesis intermediate for the compound of formula (I).

15. Use of a compound of formula (V) according to claim 1 or 14 in the synthesis of the compound of formula (I).

16. Compound of formula (V') according to claim 1, for use as a synthesis intermediate for the compound of formula (I).

17. Compound of formula (V') according to claim 16, which is 4-(3,3-diethoxypropoxy)benzamide.

18. Use of a compound of formula (V') according to claim 16 or 17 in the synthesis of the compound of formula (I).

19. Use of a compound of formula (V") according to claim 1 in the synthesis of the compound of formula (I).

20. Crystalline form I of the compound of formula (I) according to claim 1: 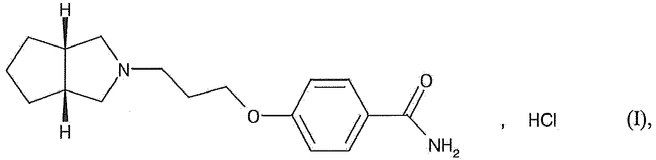 **characterised by** an X-ray powder diffraction diagram having the following diffraction lines (Bragg's angle 2 theta, expressed in degrees ±0.2°): 16.97°, 17.84°, 18.90°, 20.32°, 23.87°, 27.10°, 27.86° and 30.34°.

21. Crystalline form I of the compound of formula (I) according to claim 20, **characterised by** the following X-ray powder diffraction diagram, measured using a PANalytical X'Pert Pro MPD diffractometer with an X'Celerator detector, and expressed in terms of line position (Bragg's angle 2 theta, expressed in degrees ±0.2°) and interplanar distance d (expressed in Å):
| **Line no.** | **Angle 2-theta (degrees)** | **Interplanar distance (Å)** |
|---|---|---|
| 1 | 16.97 | 5.219 |
| 2 | 17.84 | 4.967 |
| 3 | 18.90 | 4.690 |
| 4 | 20.32 | 4.366 |
| 5 | 23.87 | 3.724 |
| 6 | 27.10 | 3.288 |
| 7 | 27.86 | 3.200 |
| 8 | 30.34 | 2.943 |

22. Crystalline form I of the compound of formula (I) according to claim 20 or 21, **characterised by** a Raman spectrum having a significant peak at the location 1606 cm⁻¹ of 1676 cm⁻¹.

23. Crystalline form I of the compound of formula (I) according to one of claims 20 to 22, **characterised by** a Raman spectrum having significant peaks at the locations 1676 cm⁻¹, 1606 cm⁻¹, 1564 cm⁻¹,1152 cm⁻¹, 830 cm⁻¹ and 296 cm⁻¹.

24. Pharmaceutical composition comprising as active ingredient crystalline form I of 4-{3-[*cis*-hexahydrocyclopenta[c]pyrrol-2(1*H*)-yl]propoxy}benzamide hydrochloride according to one of claims 20 to 23, in combination with one or more pharmaceutically acceptable, non-toxic, inert carriers.

25. Pharmaceutical composition according to claim 24 for use in the treatment of cognitive and psycho-behavioural disorders associated with cerebral ageing and with neurodegenerative diseases, and also in the treatment of mood disorders, attention-deficit hyperactivity syndrome, obesity and pain.

26. Pharmaceutical composition according to claim 24 for use in the treatment of cognitive and psycho-behavioural disorders associated with Alzheimer's disease, Parkinson's disease, Pick's disease, Korsakoff's disease, Lewy body dementias, frontal and subcortical dementias, frontotemporal dementias and vascular dementias.

27. Use of crystalline form I of 4-{3-[*cis*-hexahydrocyclopenta[*c*]pyrrol-2(1*H*)-yl]-propoxy}benzamide hydrochloride according to one of claims 20 to 23 in the manufacture of a medicament for use in the treatment of disorders of the histaminergic system.

28. Use of crystalline form I of 4-{3-[*cis*-hexahydrocyclopenta[*c*]pyrrol-2(1*H*)-yl]-propoxy}benzamide hydrochloride according to claim 27 in the manufacture of a medicament intended for the treatment of cognitive and psycho-behavioural disorders associated with cerebral ageing and with neurodegenerative diseases, and also for the treatment of mood disorders, attention-deficit hyperactivity syndrome, obesity and pain.

29. Use of crystalline form I of 4-{3-[*cis*-hexahydrocyclopenta[*c*]pyrrol-2(1*H*)-yl]-propoxy}benzamide hydrochloride according to claim 27 in the manufacture of a medicament intended for the treatment of cognitive and psycho-behavioural disorders associated with Alzheimer's disease, Parkinson's disease, Pick's disease, Korsakoff's disease, Lewy body dementias, frontal and subcortical dementias, frontotemporal dementias and vascular dementias.

30. Crystalline form I of 4-{3-[*cis*-hexahydrocyclopenta[*c*]pyrrol-2(1*H*)-yl]propoxy}-benzamide hydrochloride according to one of claims 20 to 23 for use in the treatment of disorders of the histaminergic system.

31. Crystalline form I of 4-13-[*cis*-hexahydrocyclopenta[*c*]pyrrol-2(1*H*)-yl]propoxyl-benzamide hydrochloride according to claim 30 for use in the treatment of cognitive and psycho-behavioural disorders associated with cerebral ageing and with neurodegenerative diseases, and also in the treatment of mood disorders, attention-deficit hyperactivity syndrome, obesity and pain.

32. Crystalline form I of 4-{3-[*cis*-hexahydrocyclopenta[c]pyrrol-2(1*H*)-yl]propoxy}-benzamide hydrochloride according to claim 30 for use in the treatment of cognitive and psycho-behavioural disorders associated with Alzheimer's disease, Parkinson's disease, Pick's disease, Korsakoff's disease, Lewy body dementias, frontal and subcortical dementias, frontotemporal dementias and vascular dementias.
